# EUROPÄISCHE PATENTSCHRIFT

(11) **EP 1 204 472 B1**
(45) Veröffentlichungstag und Bekanntmachung des Hinweises auf die Patenterteilung: **29.01.2003**
(21) Anmeldenummer: 00958367.5
(22) Anmeldetag: 03.08.2000
(51) Int. Cl.: B01J 19/18, B01J 19/26, B01J 14/00, C07C 67/08, C07C 69/54

(54) **VERFAHREN ZUM HERSTELLEN VON ESTERN AUS UNGESÄTTIGTEN CARBONSÄUREN UND MEHRWERTIGEN ALKOHOLEN**
METHOD FOR PRODUCING ESTERS FROM UNSATURATED CARBOXYLIC ACIDS AND POLYHYDRIC ALCOHOLS
PROCEDE DE PRODUCTION D'ESTERS A PARTIR D'ACIDES CARBOXYLIQUES NON SATURES ET D'ALCOOLS POLYVALENTS

(30) Priorität: 11.08.1999 DE 19937911
(43) Veröffentlichungstag der Anmeldung: 15.05.2002
(73) Patentinhaber: Cognis Deutschland GmbH & Co. KG, 40589 Düsseldorf (DE)
(72) Erfinder: FIES, Matthias, D-47800 Krefeld (DE); STALBERG, Theo, D-40789 Monheim (DE); RÖSSLER, Harald, D-40593 Düsseldorf (DE); GUTSCHE, Bernhard, D-40724 Hilden (DE)
(86) Internationale Anmeldenummer: EP0007496
(87) Internationale Veröffentlichungsnummer: WO01012315

(56) Entgegenhaltungen:
- EP-A- 0 713 857
- EP-A- 0 850 954
- EP-A- 0 916 643
- US-A- 5 883 288

## Beschreibung

### Gebiet der Erfindung

Die Erfindung betrifft ein Verfahren zum Herstellen von Estern aus ungesättigten Carbonsäuren mit einem höheren Siedepunkt als Wasser, insbesondere Acrylsäure oder Methacrylsäure, und mehrwertigen Alkoholen in einem Reaktor, wobei das flüssige Reaktionsgemisch Polymerisationsinhibitoren enthält und wobei man das entstehende Reaktionswasser im dampfförmigen Zustand zumindest teilweise abtrennt.

### Stand der Technik

(Meth)acrylsäureester mehrwertiger Alkohole, insbesondere aus der Gruppe der zwei- bis vierwertigen aliphatischen gesättigten Alkohole und deren Oxalkylierungsprodukte, finden zunehmende Bedeutung als hochreaktive Bestandteile in strahlenhärtenden Systemen. Solche polyfunktionellen (Meth)acryl-säureester können beispielsweise als Lackrohstoffe für die Elektronenstrahlhärtung oder als Bestandteil von UV-Lichthärtenden Druckfarben oder entsprechenden Überzugslacken, Spachtel-, Form- oder Vergußmassen oder auch in Klebstoffen, insbesondere anaerob härtenden Klebstoffen, Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)acrylsäureester der hier-betroffenen Art scheidet in aller Regel auf Grund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen, beispielsweise Verfärbungen, auslösen. Es kann weiterhin wünschenswert sein, bei der Veresterung nicht etwa nur das flüssige Reaktionsprodukt vor unerwünschten Polymerisationsreaktionen zu schützen, sondern auch eine hinreichende Inhibierung des gesamten Reaktionsraumes, und zwar sowohl des Gasinnenraumes wie der Wandflächen, die mit diesem Gasinnenraum in Kontakt stehen, sicherzustellen. Begegnet wird damit der Gefahr unerwünschter Polymerisatbildung, beispielsweise an solchen nicht geschützten Wandbereichen, wobei das Abwaschen solcher Polymerisate in das Reaktionsprodukt wiederum zu einer unerwünschten Viskositätssteigerung im Endprodukt bzw. zu unerwünschten unlöslichen Partikeln führt.

Die Erfindung ist jedoch nicht auf die Herstellung von (Meth)acrylsäureester beschränkt, sondern bezieht sich auf beliebige Ester aus ungesättigten Carbonsäuren mit einem höheren Siedepunkt als Wasser und mehrwertigen Alkoholen.

Das Verfahren der eingangs genannten Art ist z.B. aus der DE 3843843 A1 bekannt. Der Reaktionsraum wird mit einem Sauerstoff enthaltenden Gasstrom durchspült, und man belädt den mit der Gasphase erfüllten Anteil des Reaktionsinnenraumes mit feinverteilten Flüssigkeitströpfchen, die den Polymerisationsinhibitor enthalten. Auf diese Weise wird nicht nur die reaktive Flüssigphase durch Polymerisationsinhibitoren wirkungsvoll stabilisiert, sondern auch der gesamte Reaktionsinnenraum vor unerwünschten Polymerisationsreaktionen geschützt. Dabei wird der gleiche Inhibitor sowohl für den Schutz der reaktiven Flüssigphase als auch für den Schutz des mit Gas bzw. Dampf gefüllten Innenraumes und der darin angeordneten Feststoff-Flächen (Innenwände, Rührwerksteile, usw.) eingesetzt. In einem weiteren Verfahren der eingangs genannten Art zieht man das bei der Reaktion entstehende Kondensationswasser aus der Gasphase des Reaktionsraumes ab. Schließlich beschreibt die **DE 3843930 A1** die bei dem eingangs genannten Verfahren vorzugsweise einzusetzenden Polymerisationsinhibitoren.

Bei der im Batch- und Semibatch-Verfahren durchgeführten sauer katalysierten Veresterung wird zur Verhinderung der Polymerisation sowohl der eingesetzten ungesättigten Carbonsäure als auch des gebildeten ungesättigten Polyolesters ein im flüssigen Reaktionsgemisch chemisch wirkendes Inhibitorsystem eingesetzt, das zum Beispiel aus einem Hydrochinonderivat in Kombination mit Luftsauerstoff besteht. Bei der Veresterung wird das entstehende Reaktionswasser zur Erzielung hoher Umsatzgrade destillativ unter reduziertem Druck aus dem Reaktionsgemisch entfernt. Bei flüchtigen ungesättigten Carbonsäuren wie Acryl- und Methacrylsäure wird dabei eine Wasser/Carbonsäuremischung mit einer Zusammensetzung entsprechend dem Phasengleichgewicht ausgeschleust. Dieses bekannte Verfahren hat bei der Veresterung flüchtiger ungesättigter Carbonsäuren zwei gravierende Nachteile:
- Erstens zwingt die Wasser-/Carbonsäureauskreisung zu einem in Bezug auf das eingesetzte Polyol hohen stöchiometrischen Überschuß an Carbonsäure, der bei drei- und vierwertigen Polyolen 40 bis 50 % betragen kann.
- Zweitens besteht bei dem nur im flüssigen Reaktionsgemisch inhibierend wirkenden Hydrochinon/Luft-System die Gefahr, daß die bei der Wasserauskreisung in der Gasphase vorliegende, nicht inhibitierte ungesättigte Carbonsäure bei der Kondensation polymerisiert und damit zu beträchtlichen Störungen im Verfahren führt.

Der Erfindung liegt daher die Aufgabe zugrunde, im eingangs genannten Verfahren den Carbonsäureüberschuß erheblich zu reduzieren, wobei eine Polymerisation von Carbonsäure beziehungsweise Ester in der Flüssig- oder Gasphase verhindert werden soll. Außerdem soll die Reaktionszeit verkürzt und eine geringere organische Belastung des Abwassers erreicht werden.

### Beschreibung der Erfindung

Gegenstand der vorliegenden Erfindung ist demnach ein Verfahren zum Herstellen von Estern aus ungesättigten Carbonsäuren mit einem höheren Siedepunkt als Wasser, insbesondere Acrylsäure oder Methacrylsäure, und mehrwertigen Alkoholen in einem Reaktor, wobei das flüssige Reaktionsgemisch Polymerisationsinhibitoren enthält und wobei man das entstehende Reaktionswasser im dampfförmigen Zustand zumindest teilweise abtrennt, welches sich dadurch auszeichnet, daß man einen Teil der Gas/Dampfphase des Reaktionsgemisches aus dem Reaktor entfernt, diese in einem Dephlegmator partiell kondensiert, dem aufsteigenden Gas-/Dampfgemisch sowie dem ablaufenden Kondensat vom Kopf des Dephlegmators her eine den Polymerisationsinhibitor enthaltende Flüssigkeit zuführt und das gesamte ablaufende Gemisch wieder in den Reaktor zurückführt.

In dem Kondensat, welches mit Carbonsäure angereichert ist, wird die Polymerisation durch Zugabe des Inhibitors verhindert, so daß dieses Kondensat problemlos wieder in das Reaktionsgemisch zurückgeführt werden kann. Durch diese Rückführung läßt sich der für die Reaktion erforderliche Carbonsäureüberschuß von 20 bis 40 % im bekannten Verfahren auf 5 bis 10 % reduzieren. Die Partialkondensation der ungesättigten Carbonsäure führt zu einer deutlich mit Wasser angereicherten Dampfphase und damit zu einer schnelleren Destillation und zu einer Verkürzung der Reaktionszeit. Die mit Wasser angereicherte Dampfphase wird nach dem Ausschleusen kondensiert, wobei das Kondensat als Abwasser entsorgt wird. Dieses Abwasser enthält im Gegensatz zum Stand der Technik einen erheblich geringeren Anteil an ungesättigten Carbonsäuren und weist daher eine erheblich geringere organische Belastung auf.

Im Rahmen der Erfindung liegt es, dem mit Carbonsäure angereicherten Kondensat eine externe Inhibitorlösung zuzuführen. Besonders vorteilhaft ist es jedoch, daß man zu diesem Zweck einen Teil des flüssigen Reaktionsgemisches abzweigt und dem aufsteigenden Gas-/Dampfgemisch sowie dem ablaufenden Kondensat zuführt. Auf diese Weise wird ein Teil des flüssigen Reaktionsgemisches im Kreis gefahren.

Die oben genannte Abkühlung der ausgeschleusten Gas-/Dampfphase des Reaktionsgemisches kann durch ein Einsprühen einer vergleichsweise kalten, den Inhibitor enthaltenden Flüssigkeit, wobei es sich insbesondere um das Reaktionsgemisch handelt, in das Brüdenrohr erreicht werden. Wie die Erfinder festgestellt haben, kann auf diese Weise, also ohne die Verwendung eines Dephtegmators, sowohl eine Trennung von Carbonsäure und Wasser sowie eine Verhinderung der Polymerisation der kondensierten Carbonsäure erreicht werden.

Bevorzugt ist es jedoch, daß man die aus dem Reaktionsgemisch entfernte Gas-/Dampfphase mittels eines Dephlegmators partiell kondensiert, wobei eine mit Carbonsäure angereicherte flüssige Phase abläuft und eine wasserreiche dampfförmige Phase den Dephlegmator verläßt und dessen Innenwände mit der den Polymerisationsinhibitor enthaltenden Flüssigkeit benetzt. Durch die Verwendung des Dephlegmators wird eine besonders effektive Trennung von Wasser und Carbonsäure erreicht. Als Dephlegmator kann insbesondere ein vertikaler Rohrbündelwärmetauscher aus Edelstahl eingesetzt werden. Durch das Besprühen der Innenwände des Dephlegmators wird eine Inhibierung unmittelbar nach der Kondensation der Carbonsäure erzielt.

Des weiteren wurde gefunden, daß die Trennung von Carbonsäure und Wasser weiter verbessert wird, wenn die den Polymerisationsinhibitor enthaltende Flüssigkeit eine Temperatur unterhalb bis maximal zur Reaktionstemperatur hat. Nach dem Abzweigen eines Teils des Reaktionsgemisches ist es daher bevorzugt, diesen Teil zu kühlen, bevor er auf die Innenwände des Dephlegmators gesprüht wird.

Damit das mit ungesättigter Carbonsäure angereicherte kondensierte Gemisch, welches in den Reaktor zurückfließt, auf keinen Fall auf der Innenseite des Reaktordeckels oder auf Einbauten im oberen Reaktorinnenbereich, zum Beispiel auf Teilen des Rührwerkes, polymerisiert, wird weiterhin vorgeschlagen, daß man das Kondensat von oben in den Reaktor zurückführt und daß man eine den Polymerisationsinhibitor enthaltende Flüssigkeit, insbesondere einen aus dem flüssigen Reaktionsgemisch abgezweigten Teil, den Innenwänden und Einbauten im oberen Bereich des Reaktors, insbesondere der Innenwand des Reaktordeckels, zuführt und insbesondere dort aufsprüht. Auf diese Weise wird die Neigung des Kondensates zur Polymerisation auf den genannten Teilen wirkungsvoll verhindert.

Die Erfindung betrifft außerdem eine Anlage zum Durchführen des genannten Verfahrens, mit einem Reaktor und einer aufgesetzten Brüdenleitung zur Wasserabtrennung aus dem Reaktionsgemisch.

Die bereits genannte erfindungsgemäße Aufgabe wird hier gelöst durch eine erste Leitung vom unteren Bereich des Reaktors zur Brüdenleitung, innerhalb der Brüdenleitung angeordnete erste Sprühdüsen am Auslaß der ersten Leitung, wobei die Sprühdüsen insbesondere entgegengesetzt zu den aufsteigenden Brüden gerichtet sind, und eine Pumpe in der ersten Leitung.

Besonders bevorzugt ist es, wenn die Brüdenleitung als ein auf den Reaktor aufgesetzter Dephlegmator ausgebildet ist und die ersten Sprühdüsen auf die Innenwände des Dephlegmators gerichtet sind.

Um die Trennung von Wasser und Carbonsäure im Kondensat weiter zu verbessern, ist außerdem ein Kühler in der ersten Leitung von Vorteil.

Zur Verhinderung von Polymerisationen im oberen inneren Reaktorbereich wird bei der genannten Anlage weiterhin vorgeschlagen daß eine zweite Leitung entweder vom unteren Bereich des Reaktors oder abgezweigt von der ersten Leitung, vorgesehen ist und daß am Auslaß der zweiten Leitung zweite Sprühdüsen angeordnet sind, die auf die Innenwände und Einbauten im oberen Reaktorbereich, insbesondere auf die Innenwand des Reaktordeckels, gerichtet sind.

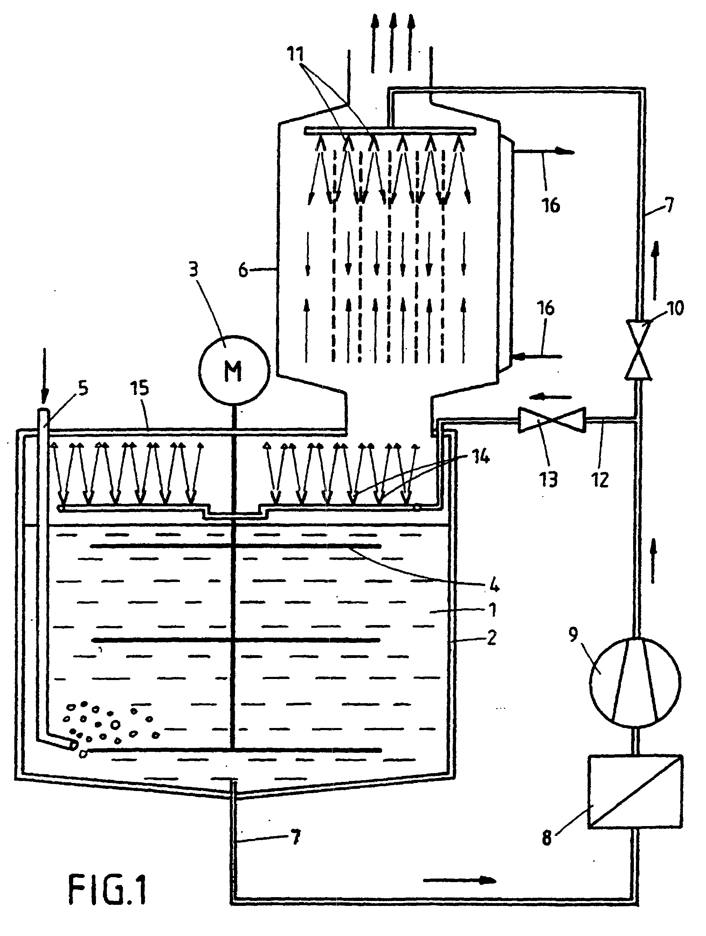

Im folgenden wird ein Ausführungsbeispiel der erfindungsgemäßen Anlage anhand der einzigen Zeichnung (Figur 1) näher beschrieben, welche eine schematische Darstellung der Anlage zeigt.

Das flüssige Reaktionsgemisch 1 im beheizten Reaktor 2 wird von einem vom Motor 3 angetriebenen Rührer 4 durchmischt. Das Reaktionsgemisch 1 enthält den Polymerisationsinhibitor, die ungesättigte Carbonsäure, den mehrwertigen Alkohol und das Reaktionsnebenprodukt Wasser sowie das Reaktionsprodukt, den Ester. Über eine Leitung 5 wird Luft von unten in das Reaktionsgemisch 1 eingeblasen.

Die oberhalb des flüssigen Reaktionsgemisches 1 befindliche Dampfphase wird über einen auf den Reaktor 2 aufgesetzten Dephlegmator 6 unter reduziertem Druck abgezogen. Dabei handelt es sich um einen vertikalen Rohrbündelwärmetauscher, wobei die Innenwände durch gestrichelte Linien angedeutet sind. Nach der Abtrennung des größten Teils der Carbonsäure wird die mit Wasser angereicherte Dampfphase nach oben abgezogen und in einem nicht dargestellten Kondensator verflüssigt. Das nur noch einen geringen Anteil von Carbonsäuren enthaltende Wasser wird als Abwasser entsorgt. Über Leitungen 16 wird der Dephlegmator 6 mittels Wasser gekühlt.

Vom Boden des Reaktors 2 führt eine erste Leitung 7 über einen Filter 8, der polymerisierte Anteile zurückhält, eine Pumpe 9 und ein Ventil 10 zu ersten Sprühdüsen 11 im oberen Bereich des Dephlegmators 6. Die ersten Sprühdüsen 11 sind nach unten auf die Innenwände des Dephlegmators 6 gerichtet.

Von der ersten Leitung 7 zweigt eine zweite Leitung 12 ab, über die das Reaktionsgemisch 1 über ein Ventil 13 zu zweiten Sprühdüsen 14 geleitet wird, die im oberen Bereich des Reaktors 2 oberhalb der Flüssigkeitsoberfläche angeordnet und nach oben auf die Innenwand des Reaktordeckels 15 gerichtet sind.

Die Anlage arbeitet wie folgt: Aus dem flüssigen, erhitzten Reaktionsgemisch steigen Wasser/Carbonsäuredämpfe in den Dephlegmator 6. Das inhibierte flüssige Reaktionsgemisch fließt gleichzeitig im Gegenstrom an den Innenwänden des Dephlegmators 6 herab und verstärkt, ähnlich wie eine unter Rückfluß betriebene Rektifizierkolonne, die Trennung von Carbonsäure und Wasser. Außerdem wird eine Polymerisation der kondensierten ungesättigten Carbonsäuren an den Dephlegmatorwänden verhindert.

Im oberen Bereich des Reaktorinnenraumes wird das inhibierte Reaktionsgemisch von den zweiten Sprühdüsen 14, die an einem Düsenring angebracht sind, insbesondere gegen die Innenwand des Reaktordeckels 15, aber auch gegen die aus dem Dephlegmator 6 am Reaktordeckel herablaufende, nicht inhibierte Carbonsäure gesprüht. Auf diese Weise wird eine Polymerisation im Deckelbereich des Reaktors verhindert.

Als Dephlegmator 6 wird ein vertikaler Rohrbündelwärmetauscher mit einer Flüssigkeitsaufgabe am Kopf über die ersten Sprühdüsen 11 eingesetzt. Das Verhältnis von Rohrdurchmesser zu Rohrlänge beträgt mindestens 0,03. Der Wärmetauscher hat eine Austauschfläche von mindestens 10 m² pro m³ Reaktorvolumen.

### Bezugszeichenliste

- 1: Reaktionsgemisch
- 2: Reaktor
- 3: Motor
- 4: Rührer
- 5: Leitung
- 6: Dephlegmator
- 7: erste Leitung
- 8: Filter
- 9: Pumpe
- 10: Ventil
- 11: erste Sprühdüsen
- 12: zweite Leitung
- 13: Ventil
- 14: zweite Sprühdüsen
- 15: Reaktordeckel
- 16: Leitung

## Patentansprüche

1. Verfahren zum Herstellen von Estern aus ungesättigten Carbonsäuren mit einem höheren Siedepunkt als Wasser, insbesondere Acrylsäure oder Methacrylsäure, und mehrwertigen Alkoholen in einem Reaktor (2), wobei das flüssige Reaktionsgemisch (1) Polymerisationsinhibitoren enthält und wobei man das entstehende Reaktionswasser im dampfförmigen Zustand zumindest teilweise abtrennt, **dadurch gekennzeichnet, daß** man einen Teil der Gas-/Dampfphase des Reaktionsgemisches (1) aus dem Reaktor (2) entfernt, diese in einem Dephlegmator (6) partiell kondensiert, dem aufsteigenden Gas-/Dampfgemisch sowie dem ablaufenden Kondensat vom Kopf des Dephlegmators (6) her eine den Polymerisationsinhibitor enthaltende Flüssigkeit zuführt und das gesamte ablaufende Gemisch wieder in den Reaktor (2) zurückführt.

2. Verfahren nach Anspruch 1, **dadurch gekennzeichnet, daß** man einen Teil des flüssigen Reaktionsgemisches (1) abzweigt und dem aufsteigenden Gas/Dampfgemisch und dem ablaufenden Kondensat zuführt.

3. Verfahren nach den Ansprüchen 1 und/oder 2, **dadurch gekennzeichnet, daß** man die aus dem Reaktionsgemisch (1) entfernte Gas-/Dampfphase mittels eines Dephlegmators (6) partiell kondensiert, wobei eine mit Carbonsäure angereicherte flüssige Phase abläuft und eine wasserreiche dampfförmige Phase den Dephlegmator (6) verläßt und dessen Innenwände mit der den Polymerisationsinhibitor enthaltenden Flüssigkeit benetzt.

4. Verfahren nach mindestens einem der Ansprüche 1 bis 3, **dadurch gekennzeichnet, daß** man eine den Polymerisationsinhibitor enthaltende Flüssigkeit einsetzt, welche eine Temperatur unterhalb bis maximal zur Reaktionstemperatur aufweist.

5. Verfahren nach mindestens einem der Ansprüche 1 bis 4, **dadurch gekennzeichnet, daß** man das Kondensat von oben in den Reaktor zurückführt und eine den Polymerisationsinhibitor enthaltende Flüssigkeit, insbesondere einen aus dem flüssigen Reaktionsgemisch (1) abgezweigten Teil, den Innenwänden und Einbauten im oberen Bereich des Reaktors (2), insbesondere der Innenwand des Reaktordeckels (15), zuführt, und insbesondere dort aufsprüht.

6. Anlage zum Durchführen eines Verfahrens nach mindestens einem der Ansprüche 1 bis 5, mit einem Reaktor (2) und einer aufgesetzten Brüdenleitung zur Wasserabtrennung aus dem Reaktionsgemisch (1), **dadurch gekennzeichnet, daß** sie eine erste Leitung (7) vom unteren Bereich des Reaktors (2) zur Brüdenleitung, innerhalb der Brüdenleitung angeordnete erste Sprühdüsen (11) am Auslaß der ersten Leitung (7), wobei die Sprühdüsen (11) insbesondere entgegengesetzt zu den aufsteigenden Brüden gerichtet sind, und eine Pumpe (9) in der ersten Leitung (7) aufweist.

7. Anlage nach Anspruch 6, **dadurch gekennzeichnet, daß** die Brüdenleitung als ein auf den Reaktor (2) aufgesetzter Dephlegmator (6) ausgebildet ist und die ersten Sprühdüsen (11) auf die Innenwände des Dephlegmators (6) gerichtet sind.

8. Anlage nach den Ansprüchen 6 undloder 7, **dadurch gekennzeichnet, daß** sie einen Kühler in der ersten Leitung (7) aufweist.

9. Anlage nach mindestens einem der Ansprüche 6 bis 8, **dadurch gekennzeichnet, daß** eine zweite Leitung (12), entweder vom unteren Bereich des Reaktors (2) oder abgezweigt von der ersten Leitung (7), vorgesehen ist und daß am Auslaß der zweiten Leitung (12) zweite Sprühdüsen (14) angeordnet sind, die auf die Innenwände und Einbauten im oberen Reaktorbereich, insbesondere auf die Innenwand des Reaktordeckels (15), gerichtet sind.

## Claims

1. A process for the production of esters of unsaturated carboxylic acids with a higher boiling point than water, more particularly acrylic acid or methacrylic acid, and polyhydric alcohols in a reactor (2), the liquid reaction mixture (1) containing polymerization inhibitors and the water of reaction formed being at least partly removed in vaporous form, **characterized in that** part of the gas/vapour phase of the reaction mixture (1) is removed from the reactor (2) and is partly condensed in a dephlegmator (6), a liquid containing the polymerization inhibitor is introduced into the ascending gas/vapor mixture and the descending condensate from the head of the dephlegmator (6) and the entire outflowing mixture is returned to the reactor (2).

2. A process as claimed in claim 1, **characterized in that** part of the liquid reaction mixture (1) is branched off and introduced into the ascending gas/vapor mixture and the descending condensate.

3. A process as claimed in claims 1 and/or 2, **characterized in that** the gas/vapor phase removed from the reaction mixture (1) is partly condensed in a dephlegmator (6), a liquid phase enriched with carboxylic acid running off and a water-rich vapor phase leaving the dephlegmator (6) and wetting its inner walls with the liquid containing the polymerization inhibitor.

4. A process as claimed in at least one of claims 1 to 3, **characterized in that** the liquid containing the polymerization inhibitor has a temperature below or at most up to the reaction temperature.

5. A process as claimed in at least one of claims 1 to 4, **characterized in that** the condensate is returned to the reactor from above and a liquid containing the polymerization inhibitor, more particularly a part branched off from the liquid reaction mixture (1), is fed to and more particularly sprayed onto the inner walls and internals in the upper part of the reactor (2), more particularly the inner wall of the reactor cover (15).

6. An installation for carrying out the process claimed in at least one of claims 1 to 5 comprising a reactor (2) surmounted by a vapor pipe for removing water from the reaction mixture (1), **characterized in that** it comprises a first pipe (7) extending from the lower part of the reactor (2) to the vapor pipe, first spray nozzles (11) arranged within the vapor pipe at the outlet of the first pipe (7), the spray nozzles (11) being directed in particular oppositely to the ascending vapors, and a pump (9) in the first pipe (7).

7. An installation as claimed in claim 6, **characterized in that** the vapor pipe is in the form of a dephlegmator (6) surmounting the reactor (2) and the first spray nozzles (11) are directed onto the inner walls of the dephlegmator (6).

8. An installation as claimed in claims 6 and/or 7, **characterized in that** it comprises a cooler in the first pipe (7).

9. An installation as claimed in at least one of claims 6 to 8, **characterized in that** a second pipe (12) - either extending from the lower part of the reactor (2) or branched off from the first pipe (7) - is provided and second spray nozzles (14) are arranged at the outlet of the second pipe (12) and are directed onto the inner walls and internals in the upper part of the reactor, more particularly onto the inner wall of the reactor cover (15).

## Revendications

1. Procédé de fabrication dans un réacteur (2) d'esters à partir d'acides carboniques insaturés présentant un point d'ébullition supérieur à celui de l'eau, en particulier l'acide acrylique ou l'acide méthacrylique, et d'alcools polyvalents, le mélange réactionnel (1) liquide contenant des inhibiteurs de polymérisation et l'eau produite par la réaction étant séparée au moins partiellement à l'état de vapeur,
**caractérisé en ce qu'**
on retire du réacteur (2) une partie de la phase gaz/vapeur du mélange réactionnel (1) et on la condense en partie dans un déphlegmateur (6), on introduit dans le mélange gaz/vapeur qui s'élève et dans le condensat s'écoulant de la tête du dephlegmateur (6) un liquide contenant l'inhibiteur de polymérisation et tout l'ensemble sortant est réintroduit dans le réacteur (2).

2. Procédé selon la revendication 1,
**caractérisé en ce qu'**
on dérive une partie du mélange réactionnel (1) et on l'introduit dans le mélange gaz/vapeur qui s'élève et dans le condensat qui s'écoule.

3. Procédé selon l'une quelconque des revendications 1 et/ou 2,
**caractérisé en ce que**
la phase gaz/vapeur retirée du mélange réactionnel (1) est condensée en partie au moyen d'un déphlegmateur (6), de sorte qu'il s'écoule une phase liquide enrichie en acide carbonique et qu'il sort du dephlegmateur (6) une phase gazeuse riche en eau, les parois internes de celui-ci étant mouillées par le liquide contenant l'inhibiteur de polymérisation.

4. Procédé selon l'une quelconque des revendications 1 à 3,
**caractérisé en ce qu'**
on introduit un liquide contenant l'inhibiteur de polymérisation, à une température au plus égale à la température de réaction.

5. Procédé selon l'une quelconque des revendications 1 à 4,
**caractérisé en ce que**
le condensat est réintroduit d'en haut dans le réacteur et on amène le liquide contenant l'inhibiteur de polymérisation, en particulier une partie issue par déviation du mélange réactionnel (1), sur les parois internes et les chicanes situées à la partie supérieures du réacteur (2) en particulier la face interne du couvercle du réacteur (15), et notamment on les pulvérise en cet endroit notamment.

6. Installation de mise en oeuvre d'un procédé selon l'une quelconque des revendications 1 à 5, utilisant un réacteur (2) avec une conduite de vapeur pour séparer l'eau du mélange réactionnel (1),
**caractérisée en ce qu'**
elle comporte une première conduite (7) reliant la zone inférieure du réacteur (2) à la conduite de vapeur, dans cette dernière des premières buses de pulvérisation (11) sont montées à la sortie de la première conduite (7) en étant notamment dirigées dans le sens opposé de celui des vapeurs qui s'élèvent et une pompe (9) est montée dans la première conduite (7).

7. Installation selon la revendication 6,
**caractérisée en ce que**
la conduite de vapeur à la forme d'un déphlegmateur (6) monté sur le réacteur (2) et les premières buses de pulvérisation (11) sont dirigées sur les parois internes du déphlegmateur (6).

8. Installation selon l'une quelconque des revendications 6 et/ou 7,
**caractérisée en ce qu'**
elle comporte un refroidisseur dans la première conduite (7).

9. Installation selon l'une quelconque des revendications 6 à 8,
**caractérisée en ce qu'**
une seconde conduite (12) part de la zone inférieure du réacteur (2) ou est dérivée sur la première conduite (7), et à la sortie de la seconde conduite (12) sont montées des secondes buses de pulvérisation (14) dirigées sur les parois internes et sur les chicanes de la zone supérieure du réacteur, en particulier la paroi interne du couvercle (15) du réacteur.
